(19) Europäisches Patentamt
European Patent Office
Office européen des brevets

(11) Veröffentlichungsnummer: **0 025 829**
**B1**

# (12) EUROPÄISCHE PATENTSCHRIFT

(45) Veröffentlichungstag der Patentschrift: **22.12.82**

(51) Int. Cl.³: **C 07 C 17/33,** C 07 C 19/02

(21) Anmeldenummer: **80104089.0**

(22) Anmeldetag: **15.07.80**

(54) **Verfahren zur Herstellung von Alkylchloriden.**

(30) Priorität: **04.08.79 DE 2931777**

(43) Veröffentlichungstag der Anmeldung: **01.04.81 Patentblatt 81/13**

(45) Bekanntmachung des Hinweises auf die Patenterteilung: **22.12.82 Patentblatt 82/51**

(84) Benannte Vertragsstaaten: **CH DE FR GB IT LI**

(56) Entgegenhaltungen:
**DE-A-2 329 036**
**DE-A-2 545 659**
**Chemical Abstracts Band 58, Nr. 12, 10. Juni 1963 Columbus, Ohio, USA K.W. BUCK et al. «Reaktions of some alkyl chloroformates» Spalten 12654h and 12655a & J. CHEM. Soc., 1963, Seiten 2217 bis 2221**
**Chemical Abstracts Band 67, Nr. 17 23. Oktober 1967 Columbus, Ohio, USA A.B. Foster et al. «Pyridine-catalyzed decarboxylation of cis- and trans-4-tert-butylcyclohexyl chloroformate» Seite 7701, Spalte 2, Abstract Nr. 81828j & Carbohyd. Res. Band 4, Nr. 4, 1967, Seiten 352 bis 354 (Eng)**

(73) Patentinhaber: **BASF Aktiengesellschaft, Carl-Bosch-Strasse 38, D-6700 Ludwigshafen (DE)**

(72) Erfinder: **Neumayr, Franz, Dr., Im Eiertal 8, D-6719 Weisenheim (DE)**
Erfinder: **Decker, Martin, Dr., Maria-Stuart-Strasse 21, D-6700 Ludwigshafen (DE)**

EP 0 025 829 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von Alkylchloriden

Diese Erfindung betrifft ein Verfahren zur Herstellung von Alkylchloriden durch Decarboxylierung von Alkylchlorformiaten.

Aus J. Chem. Soc. Band 1971, Seiten 747 bis 751 ist bekannt, dass Pyridin die Decarboxylierung von Chlorformiaten katalysiert. Die Reaktion verläuft über eine definierte salzartige Zwischenstufe. Für die Herstellung von Alkylchloriden ist dieses Verfahren wenig geeignet, da als Nebenprodukt grössere Mengen an Pyridinhydrochlorid gebildet werden. Vorteilhafter lassen sich Alkylchloride aus den entsprechenden Alkylchlorformiaten herstellen, wenn man die Decarboxylierung in Carbonsäureamiden, insbesondere N-Methylpyrrolidon, vornimmt. Bei der Durchführung dieses in der DE-OS 25 45 659 beschriebenen Verfahrens stellte sich jedoch heraus, dass im Kühler hinter dem Reaktor Salzablagerungen auftreten, die die Einführung des Verfahrens in die Technik unmöglich machen.

Aus Chemical Reviews, Vol. 64 (1964), Seite 661 ist bekannt, dass auch Trialkylamine mit Chlorformiaten reagieren. Bei dieser Umsetzung entstehen jedoch als Hauptprodukt die entsprechenden Urethane.

Es wurde nun ein vorteilhaftes Verfahren zur Herstellung von gegebenenfalls substituierten Alkylchloriden, in denen der Alkylrest, der auch aus durch Sauerstoff unterbrochenen Alkylresten bestehen kann, als Substituenten Alkylgruppen, Arylgruppen oder Halogenatome enthalten kann, durch Decarboxylierung der entsprechenden Alkylchlorformiate bei höherer Temperatur gefunden, bei dem man die Decarboxylierung bei Temperaturen von 90 bis 170 °C und in Gegenwart eines Trialkylamin-Hydrochlorids vornimmt.

Nach dem erfindungsgemässen Verfahren werden die Alkylchloride auf besonders einfache Weise hergestellt, ohne dass bei der technischen Durchführung die genannten Schwierigkeiten auftreten.

Die als Ausgangstoffe zu verwendenden Alkylchlorformiate sind z.B. Verbindungen der Formel $R(OCOCl)_n$, in der R ein Alkylrest, der auch aus durch Sauerstoff unterbrochenen Alkylresten bestehen kann, bedeutet, der als Substituenten Alkylgruppen, Arylgruppen oder Halogenatome enthalten kann und n für die Zahl 1 oder 2 steht. Nach der erfindungsgemässen Umsetzung entstehen daraus die entsprechenden Alkylchloride der Formel $R(Cl)_n$.

Beispielsweise seien folgende Reste R genannt: Propyl, Butyl, Pentyl, Amyl, Phenylpropyl, oder die zweiwertigen Reste der Formeln $-(CH_2)_4-$ und $-C_2H_4-O-C_2H_4-$.

Die Alkylchlorformiate sind auf bekannte Weise, z.B. durch Umsetzung der entsprechenden Alkohole, mit Phosgen erhältlich.

Als Trialkylamine kommen z.B. solche in Betracht, deren Alkylgruppen 3 bis 12 C-Atome enthalten. Besonders gut geeignet sind die Hydrochloride von Tri-n-propylamin und von Tri-n-butylamin.

Das neue Verfahren wird z.B. so durchgeführt, dass man das Trialkylamin-Hydrochlorid vorlegt, auf die Reaktionstemperatur von 90 bis 170 °C erhitzt und dann das Alkylchlorformiat zugibt. Bevorzugt sind Temperaturen von 110 bis 150 °C, insbesondere von 125 bis 135 °C. Bei diesen Temperaturen läuft die Reaktion mit genügend hoher Geschwindigkeit ab. Sind die gebildeten Alkylchloride bei den Reaktionstemperaturen flüchtig, so entweicht das entstehende Alkylchlorid im Masse der Zudosierung des Alkylchlorformiates dampfförmig und kann hierbei unmittelbar isoliert werden. Die Menge des vorgelegten Trialkylamin-Hydrochlorids ist nicht kritisch, da nur geringe Verluste dieses Katalysators beobachtet werden. Zweckmässigerweise legt man die zur Herstellung einer Schmelze ausreichende Menge des Amin-Hydrochlorids vor, wobei es zur Bildung eines homogenen Gemisches vorteilhaft sein kann, geringe Mengen Lösungsmittel, wie Wasser, oder das herzustellende Produkt zuzugeben.

Sind die bei der Synthese gebildeten Alkylchloride bei den Reaktionstemperaturen nicht flüchtig, so stellt man die Konzentration an den Trialkylamin-Hydrochloriden im Reaktionsgemisch zweckmässigerweise so ein, dass sie nicht unter 3 Gew.-% abfällt. Man arbeitet das Reaktionsgemisch nach Beendigung der Reaktion zweckmässig durch Destillation auf. Man kann anstelle der Alkylchlorformiate auch die entsprechenden Alkohole einsetzen und zum Reaktionsgemisch Phosgen geben. In diesem Falle werden zunächst die Alkylchlorformiate gebildet, die dann sofort unter Decarboxylierung die Alkylchloride bilden.

Beispiel 1

In einem emailierten Kessel mit 8 m³ Inhalt werden 1 000 kg Tri-n-propylamin vorgelegt. Man gibt 40 l Wasser hinzu und leitet in die Mischung gasförmigen Chlorwasserstoff bis zur Sättigung unter Kühlung ein. Man erhitzt die Mischung auf 130 °C und leitet in die Schmelze unter Rühren 200 l/h n-Butylchlorformiat ein. Dabei entweicht das dabei gebildete n-Butylchlorid sofort dampfförmig. Das in einem nachgeschalteten Kühler kondensierte rohe n-Butylchlorid wird mit Wasser gewaschen und destilliert. Aus 13 680 kg n-Butylchlorformiat erhält man 9 280 kg n-Butylchlorid, das noch einen Gehalt von 0,3% Tripropylaminhydrochlorid aufweist.

Beispiel 2

In einem mit Rührer, Rückflusskühler, Thermometer und Tropftrichter ausgerüsteten 4-Liter-Vierhalskolben werden 100 g Tributylamin vorgelegt. Dann leitet man durch ein Gaseinleitrohr Chlorwasserstoff bis zur Sättigung ein. Der so gebildete Katalysator wird anschliessend mit einem Heizmantel auf 140 °C aufgeheizt. Dann werden insgesamt 3 880 g Diglykol-bis-chlorformiat

in die Reaktionsmischung zudosiert. Nach beendeter Reaktion wird das Reaktionsgemisch destilliert. Man erhält 2 280 g 2,2'-Di-chlordiethylether, d.h. etwa 95% d.The.. Der Gehalt an Tributylaminhydrochlorid im Destillat beträgt 0,1%.

Beispiel 3

In einem mit Thermometer, Tropftrichter und Gaseinleitungsrohr sowie einem Intensivkühler ausgerüsteten 1-Literkolben werden 100 g 1,4-Dichlorbutan und 50 g Tri-n-butylamin vorgelegt. In diese Mischung leitet man gasförmigen Chlorwasserstoff bis zur Sättigung ein. Dann wird die Mischung auf 145 °C erhitzt und bei dieser Temperatur unter Rühren gemeinsam 45 g Butandiol und 110 g Phosgen je Stunde zudosiert. Nach Zugabe von 540 g Butandiol und 1320 g Phosgen wurde das überschüssige Phosgen abdestilliert. Bei der anschliessenden Reindestillation werden 641 g 1,4-Dichlorbutan entsprechend 82% d.Th. erhalten.

**Patentansprüche**

1. Verfahren zur Herstellung von gegebenenfalls substituierten Alkylchloriden, in denen der Alkylrest, der auch aus durch Sauerstoff unterbrochenen Alkylresten bestehen kann, als Substituenten Alkylgruppen, Arylgruppen oder Halogenatome enthalten kann, durch Decarboxylierung der entsprechenden Alkylchlorformiate bei höherer Temperatur, dadurch gekennzeichnet, dass man die Decarboxylierung bei Temperaturen von 90 bis 170 °C und in Gegenwart eines Trialkylamin-Hydrochlorids vornimmt.

2. Verfahren gemäss Anspruch 1, dadurch gekennzeichnet, dass man Alkylchloride der Formel

$$R(Cl)_n,$$

in der R ein Alkylrest, der auch aus durch Sauerstoff unterbrochenen Alkylresten bestehen kann, bedeutet, der als Substituenten Alkylgruppen, Arylgruppen oder Halogenatome enthalten kann und n für die Zahl 1 oder 2 steht, durch Decarboxylierung aus den entsprechenden Alkylchlorformiaten der allgemeinen Formel

$$R(OCOCl)_n$$

herstellt.

3. Verfahren nach Anspruch 1, dadurch gekennzeichnet, dass man die Decarboxylierung bei Temperaturen von 110 bis 150 °C vornimmt.

4. Verfahren nach Anspruch 1, dadurch gekennzeichnet, dass man die Decarboxylierung in Gegenwart von Tri-n-propylamin-Hydrochlorid oder von Tri-n-butyl-amin-Hydrochlorid vornimmt.

**Revendications**

1. Procédé de préparation de chlorures d'alkyle éventuellement substitués dans lesquels le groupe alkyle, qui peut également consister en un groupe alkyle interrompu par l'oxygène, peut porter en tant que substituants des groupes alkyle, des groupes aryle ou des atomes d'halogènes, par décarboxylation des chloroformiates d'alkyle correspondants à chaud, caractérisé en ce qu'on effectue la décarboxylation à des températures de 90 à 170 °C et en présence d'un chlorhydrate de trialkylamine.

2. Procédé selon la revendication 1, caractérisé en ce que l'on prépare des chlorures d'alkyle de formule

$$R(Cl)_n,$$

dans laquelle R représente un groupe alkyle qui peut également consister en un groupe alkyle interrompu par l'oxygène, et qui peut porter en tant que substituant des groupes alkyle, des groupes aryle ou des atoms d'halogènes, et n est égal à 1 ou 2, par décarboxylation à partir des chloroformiates d'alkyle correspondants de formule générale

$$R(OCOCl)_n$$

3. Procédé selon la revendication 1, caractérisé en ce que la décarboxylation est effectuée à des températures de 110 à 150 °C.

4. Procédé selon la revendication 1, caractérisé en ce que la décarboxylation est effectuée en présence de chlorhydrate de tri-n-propylamine ou de chlorhydrate de tri-n-butyl-amine.

**Claims**

1. Process for the preparation of optionally substituted alkyl chlorides, in which the alkyl radical, which can also consist of alkyl radicals interrupted by oxygen, may contain alkyl groups, aryl groups or halogen atoms as substituents, by decarboxylating the corresponding alkyl chloroformates at an elevated temperature, wherein the decarboxylation is carried out at temperaturs of 90 to 170 °C and in the presence of a trialkylamine hydrochloride.

2. Process according to claim 1, wherein alkyl chlorides of the formula

$$R(Cl)_n$$

where R denotes an alkyl radical, which can also consist of alkyl radicals interrupted by oxygen, and which may contain alkyl groups, aryl groups or halogen atoms as substituents, and n is the number 1 or 2, are prepared from the corresponding alkyl chloroformates of the general formula

$$R(OCOCl)_n$$

by decarboxylation.

3. Process according to claim 1, wherein the decarboxylation is carried out at temperatures of 110 to 150 °C.

4. Process according to claim 1, wherein the decarboxylation is carried out in the presence of tri-n-propylamine hydrochloride or of tri-n-butyl-amine hydrochloride.